# EUROPEAN PATENT APPLICATION

(11) **EP 4 086 262 A1**
(43) Date of publication of application: **09.11.2022**
(21) Application number: 20909068.7
(22) Date of filing: 22.12.2020
(51) Int. Cl.: C07D 491/147, G01N 21/64

(54) **REAL-TIME FLUORESCENCE IMAGING SENSOR FOR MEASURING GLUTATHIONE IN ENDOPLASMIC RETICULUM AND METHOD USING SAME**

(30) Priority: 30.12.2019 KR 20190178260
(71) Applicant: Cell2in, Inc., Seoul 03080 (KR)
(72) Inventor: KANG, Heun Soo, Seoul 06272 (KR); KIM, Hye Mi, Seoul 01054 (KR); YANG, Gwang Mo, Seoul 05010 (KR); KIM, Yeji, Seoul 08799 (KR); SHIN, Ji Woong, Seoul 03438 (KR); KANG, Hye Won, Seoul 08303 (KR); KIM, Yong Hwan, Anyang-si Gyeonggi-do 14055 (KR); CHOI, Ki Hang, Seoul 02027 (KR); MIN, Su-Hyeon, Seoul 02585 (KR)
(74) Representative: Müller, Christian Stefan Gerd
(86) International application number: PCT/KR2020/018859
(87) International publication number: WO 2021/137500

(57) **Abstract**

The present invention relates to a real-time fluorescence imaging sensor for measuring glutathione in the endoplasmic reticulum (ER) and a method for producing same. More specifically, the present invention relates to a new compound for measuring glutathione in the endoplasmic reticulum (ER), a method for producing the new compound, a real-time imaging sensor for measuring glutathione in the endoplasmic reticulum (ER) comprising the new compound, a method for producing same, and a method for measuring glutathione in the endoplasmic reticulum (ER) by using the imaging sensor.

## Description

### Technical Field

The present invention relates to a real-time fluorescence imaging sensor for measuring glutathione in the endoplasmic reticulum (ER) and a method using the same. More specifically, the present invention relates to a novel compound for measuring glutathione in the endoplasmic reticulum (ER) and a method of measuring glutathione in the endoplasmic reticulum (ER) using the novel compound.

### Background Art

The human body maintains homeostasis by properly eliminating reactive oxygen species (ROS) through the action of the antioxidant systems. However, when the balance between ROS production and the action of the antioxidant systems is destroyed, oxidative stress increases, which has recently received attention as the primary common cause of development of aging, age-related degenerative diseases, including degenerative arthritis, cataract and Alzheimer's disease, various cancers, fibrosis diseases, as well as metabolic syndromes, including diabetes, obesity and cardiovascular diseases. The ROSs are unstable and highly reactive molecules that oxidize biological molecules to cause biochemical and physiological damage, which is one of the major mechanisms of aging. Thus, not only the degree of oxidation in the human body, but also the degree of antioxidation or antioxidant activity can be used as major biomarkers for measuring biological age.

Meanwhile, mesenchymal stem cells are multipotent stem cells derived from various adult cells such as bone marrow, umbilical cord blood, placenta (or placental tissue cells), and fat (or adipose tissue cells). For example, mesenchymal stem cells derived from bone marrow have multipotency capable of differentiating into adipose tissue, bone/cartilage tissue, and muscle tissue, and thus various studies have been conducted for development of cell therapy products using the mesenchymal stem cells.

However, stem cells, which are the main components of cell therapy products, lose their multipotency and tissue regeneration ability in culture processes after isolation and are prone to aging, and this risk further increases when these cells undergo several passages to obtain a large amount of cells corresponding to a therapeutically effective amount. In addition, stem cells obtained from tissues are very small in amount, and for the use of these cells, a large amount of cells are required, and thus culture is performed to increase the number of stem cells. In recent years, as a method of managing the quality of stem cells by measuring the antioxidant activity of stem cells in relation to the quality of stem cells, a method of measuring intracellular antioxidant activity has been disclosed.

However, studies on a method of screening high-quality stem cells having high activity by measuring the antioxidant activity of stem cells are still insufficient. Thus, in order to increase the efficiency of use of stem cells, which are cell therapy product resources having a high scarcity value, it is necessary to develop a composition for measuring antioxidant activity, which is require to screen highly active stem cells.

In addition, detection and identification of a thiol-containing substance in a biological sample is very important in measurement of the antioxidant activity of cells including stem cells as described above. Accordingly, fluorescence methods of effectively detecting thiols in living cells without disrupting cells have been developed, but a substance for measuring antioxidant activity by measuring thiols from various sources in the endoplasmic reticulum is required.

### DISCLOSURE

### Technical Problem

The present inventors have found, by using a novel Endoplasmic Reticulum Fluorescent Real-time SH group-Tracer (ER-FT) according to the present invention, that the fluorescence intensity increases or decreases continuously, ratiometrically or reversibly depending on the level of thiols in the endoplasmic reticulum (ER), and have found that the ER-FT may be effectively used as a highly sensitive biosensor for real-time quantitative or qualitative detection of the level of thiols in the endoplasmic reticulum (ER) in living cells, thereby completing the present invention.

Therefore, an object of the present invention is to provide endoplasmic reticulum fluorescent real-time SH group-tracers (ER-FTs) represented by Formulas IV to VII.

Another object of the present invention is to provide a composition for detecting thiol in the endoplasmic reticulum (ER), the composition containing an endoplasmic reticulum fluorescent real-time SH group-tracer (ER-FT).

Still another object of the present invention is to provide a method of screening an agent for increasing or inhibiting thiol in the endoplasmic reticulum (ER) in living cells by using ER-FT.

Objects and advantage of the present invention will be more apparent from the following detailed description of the invention and the appended claims.

However, objects to be achieved by the present invention are not limited to the above-mentioned objects, and other objects not mentioned herein may be clearly understood by those of ordinary skill in the art from the following description.

### Technical Solution

Hereinafter, various embodiments described herein will be described with reference to the drawings. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, well-known processes and manufacturing techniques have not been described in particular detail in order not to unnecessarily obscure the present invention. Reference throughout this specification to "one embodiment" or "an embodiment" means that a particular feature, configuration, composition, or characteristic described in connection with the embodiment is included in at least one embodiment of the invention. Thus, the appearances of the phrase "in one embodiment" or "an embodiment" in various places throughout this specification are not necessarily referring to the same embodiment of the invention. Furthermore, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present invention pertains.

As used herein, the term "ratiometric" means that output is directly proportional to input. Specifically, in one embodiment of the present invention, the term "ratiometric" means that the fluorescence intensity of the composition of the present invention increases or decreases in direct proportion to the input of thiols.

As used herein, the term "detection" means measuring the presence or level of chemical species or biological substances in a sample.

As used herein, the term "reversible" means a state in which a mixture of a reactant and a product in a chemical reaction can produce an equilibrated mixture. More specifically, the term "reversible" means that a compound represented by Formula I in this specification can react reversibly with thiols in an equilibrium state in a forward or reverse direction depending on the amount of the thiols.

As used herein, the term "thiol" means an organic compound containing a carbon-bonded sulfhydryl group. The term "thiol group" is used interchangeably with the term "sulfhydryl group".

As used herein, the term "cells" means a structural or functional unit that constitutes a living body. For the purpose of the present invention, cells include, without limitation, cells undergoing cellular senescence.

As used herein, the term "cellular senescence" refers to a series of processes including degeneration of cellular characteristics and functions until the time of cell death or proliferation arrest. The degeneration of cellular characteristics and functions may be irreversible. In addition, cellular senescence may include, but is not limited thereto, decreased cellular proliferative capacity, loss of pluripotency and tissue regeneration ability, lipofuscin accumulation, increased β-galactosidase activity, increased mitochondrial reactive oxygen species production, or combinations thereof, compared to normal cells or organisms, or may show processes causing them. Cellular or organismal senescence may include decreased autophagy activity or decreased mitochondrial membrane potential, or may further include a process causing the same. Young cells or organisms may show increased cellular proliferation ability, decreased lipofuscin accumulation, decreased β-galactosidase activity, or combinations thereof, compared to normal cells or organisms. For example, senescent cells may be cells having a doubling time that increased by 2 times or more, 3 times or more, 4 times or more, 5 times or more, 6 times or more, 7 times or more, 9 times or more, 10 times or more, 50 times or more, or 100 times or more compared to the doubling times at passage 2.

In the present invention, the cellular senescence can be induced early when cells are actively divided both *in vitro* and *in vivo* through the application of endogenous and exogenous stimuli that are associated with proliferative stress and/or evoke DNA damage. Such stimuli include the aberrant expression and/or activation of oncogenes, direct DNA damage caused by exposure to ionizing radiation, reactive oxygen species, chemotherapeutic drugs, and an increase in the number of passages due to cell passage. Consequently, the cellular senescence can positively affect cellular stress responses, cancer development and treatment outcomes, and stem cell treatment outcomes.

As used herein, the term "degree of cellular senescence" may refer to a quantitative or qualitative measurement of the degree of cellular senescence.

As used herein, the term "antioxidant activity" refers to the ability of an antioxidant to prevent oxidation, which can be measured simply by placing fat in a closed container containing oxygen and measuring the oxygen consumption rate. However, the measurement of antioxidant activity of antioxidants in the biochemistry of organisms is becoming important.

As used herein, the term "antioxidant" is also called an antioxidant substance or an oxidation preventing agent and refers to a substance that prevents oxidation. Here, antioxidation means inhibition of oxidation. This term is a concept that appears mainly when describing the cellular senescence process and the prevention of cellular senescence, and cellular senescence means oxidation of cells. Oxygen entering the body through respiration has a beneficial effect on the body, but reactive oxygen species are produced in this process. Excessive reactive oxygen species cause exposure of oxygen to an unstable state, which adversely affects the animal's body. Thus, proper maintenance of reactive oxygen species may prevent cellular oxidation and cellular senescence, without being limited thereto.

Examples of the antioxidant of the present invention include, but are not limited to, extracellular and intracellular substances such as carotenoids (beta-carotene, lycopene, and lutein), flavonoids (anthocyanin, catechin, resveratrol, and proanthocyanidins), isoflavones (genistein, and daidzein), vitamins, minerals, glutathione, coenzyme Q-10, catalase, superoxide dismutase, glutathione-dependent peroxidase, and peroxiredoxin.

As used herein, the term "oxidative stress" refers to stress that occurs as the antioxidant activity in a living body (cells) decreases with an increase in the oxidation rate (the proportion of reactive oxygen species) caused by the destruction of the balance between the oxidizing substances generated in the living body (or cells) and antioxidants corresponding thereto, and the terms "oxidative damage" or "oxidation degree" may be used to collectively refer to various damage (gene damage, cellular metabolic abnormalities, etc.) in a living body or cells, which are caused by such oxidative stress, without being limited thereto.

In this specification, unless otherwise specified, oxidative damage substances (or oxidative damage inducing substances) and oxidative stress substances (or oxidative stress inducing substances) are used in the same sense.

According to the present invention, it is possible to measure the presence and concentration of an oxidative stress-inducing substance to be detected or oxidative damage substance by measuring oxidative stress.

According to one embodiment of the present invention, the present invention provides a composition for detecting thiol in the endoplasmic reticulum (ER), the composition containing a compound represented by the following Formula I or a salt thereof:

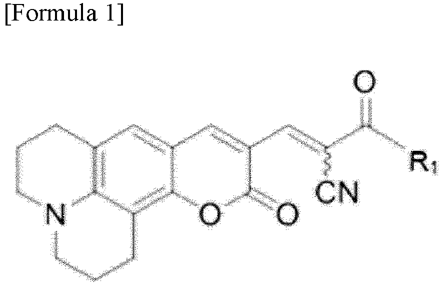

wherein R₁ is a 3- to 7-membered heterocycloalkyl ring containing at least one N atom, an R₂ substituent is bonded to the heterocycloalkyl, R₂ is an amide group, and R₂ is -(C(=O)NH)-R₃, wherein R₃ may be -(CH₂)ₘ-R₄, -(CH₂)ₘ-R₅, -(CH₂)ₙ-(CH₂OCH₂)ₚ-(CH₂)_{q}-R₄, or -(CH₂)ₙ-(CH₂OCH₂)ₚ-(CH₂)_{q}-R₅, wherein m is an integer ranging from 1 to 6, n, p and q are each independently an integer ranging from 1 to 4, R₄ is a substituent represented by the following Formula II, and R₅ is a substituent represented by the following Formula III:

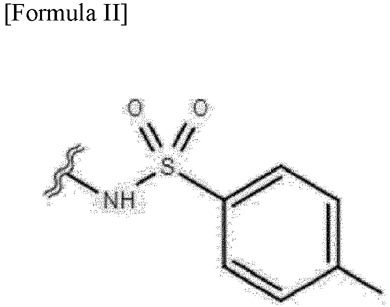

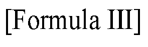

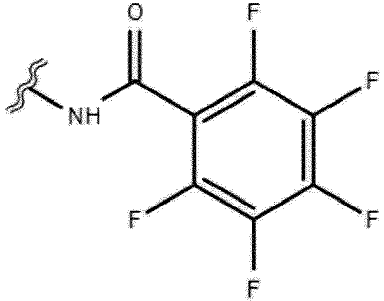

The present inventors have made extensive efforts to develop a highly sensitive biosensor for quantitatively or qualitatively detecting the level of thiols in the intracellular endoplasmic reticulum (ER) in real time. As a result, the present inventors have found that the fluorescence intensity of an ER-FT (Endoplasmic Reticulum Fluorescent Real-time SH group-Tracer) of the present invention, which is represented by Formula I, increases or decreases continuously, ratiometrically and reversibly depending on the level of thiols in the intracellular endoplasmic reticulum (ER) and that the ER-FT may be effectively used as a highly sensitive biosensor for quantitatively or qualitatively detecting the level of thiols in the intracellular endoplasmic reticulum (ER) in real time.

In the present invention, the fluorescence intensity may increase or decrease at an emission wavelength ranging from 430 nm to 680 nm.

As used herein, the term "Endoplasmic Reticulum Fluorescent Real-time SH group-Tracer (ER-FresH, ER-FT)" means the compound represented by Formula I, which is a coumarin derivative having a cyanoacrylamide electrophile and is used as a fluorescent substance for detection of thiols in the endoplasmic reticulum (ER) in the present invention.

In one embodiment of the present invention, the endoplasmic reticulum (ER) of the present invention is contained in a living cell. The composition of the present invention for measuring the level of thiols in the endoplasmic reticulum (ER) is characterized in that it can measure not only the level of thiols in the endoplasmic reticulum (ER) isolated from the cell, but also the level of thiols in the endoplasmic reticulum (ER) contained in a cell. In particular, the composition may specifically detect the level of thiols in the endoplasmic reticulum (ER) in living cells.

In one embodiment of the present invention, R₁ in the present invention is a 6-membered heterocycloalkyl ring containing 1 or 2 N atoms, and an R₂ substituent is bonded to the heterocycloalkyl, and R₂ is a substituted or unsubstituted amide group. As used herein, the term "6-membered ring" included in "6-membered heterocycloalkyl ring" refers to a single 6-membered ring, which is a monocyclic compound, rather than a cyclic compound containing a plurality of rings fused, such as a bicyclic compound or a spiro compound, and the term "heterocycloalkyl" refers to a non-aromatic cyclic alkyl in which at least one of carbon atoms included in the ring is substituted with a heteroatom, for example, nitrogen, oxygen or sulfur. In this embodiment, R₁ is a 6-membered heterocycloalkyl ring containing one or two nitrogen atoms as heteroatoms in the ring.

In the present invention, the compound represented by Formula I is any one or more of compounds represented by the following Formulas IV to VII:

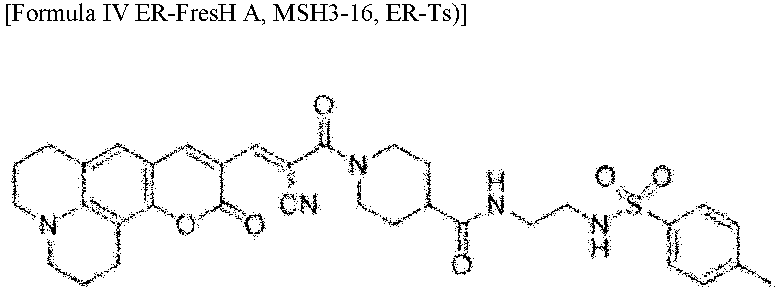

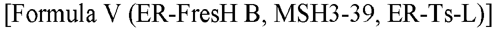

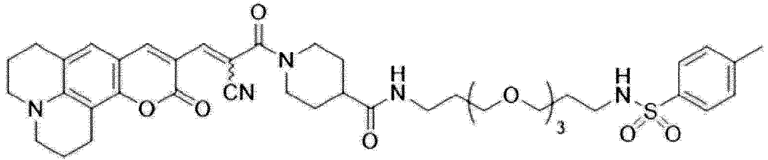

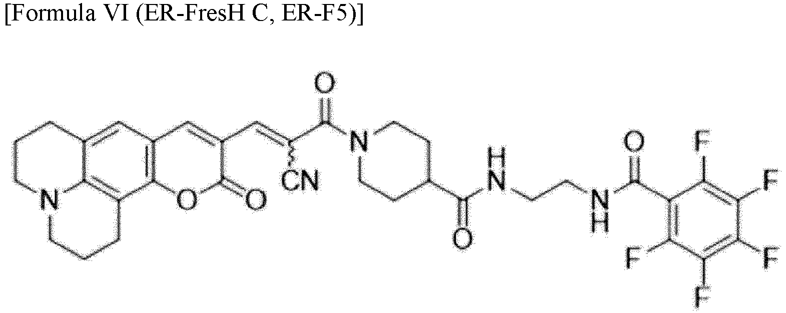

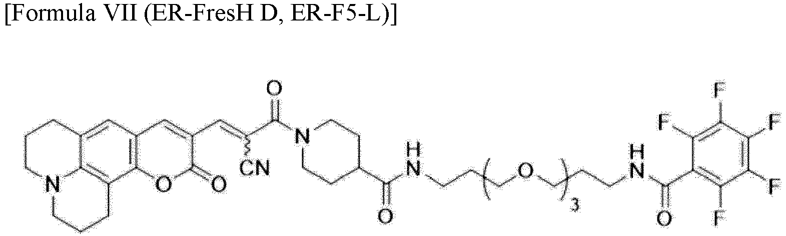

The amount of thiols binding to the compound (ER-FT) represented by Formula I according to the present invention, preferably the compound represented by any one of Formulas IV to VII, may increase as the amount of thiols in the endoplasmic reticulum (ER) in living cells increases.

In the present invention, the compound represented by Formula I, preferably the compound represented by any one of Formulas IV to VII, may be a compound represented by Formula I or a pharmaceutically acceptable salt thereof, which exhibits a maximum emission wavelength at 550 to 680 nm in a free state and exhibits a maximum emission wavelength at 430 to 550 nm in a thiol-bound state.

In the present invention, the compound represented by Formula I, preferably the compound represented by any one of Formulas IV to VII, exhibits a maximum emission wavelength at 550 to 650, 550 to 620, 550 to 600, 570 to 590 or 580 nm in a free state.

In the present invention, the compound represented by Formula I, preferably the compound represented by any one of Formulas IV to VII, exhibits a maximum emission wavelength at 450 to 550, 470 to 550, 470 to 530, 490 to 530, 500 to 520 or 510 nm in a thiol-bound state.

In the present invention, the compound represented by Formula I or a pharmaceutically acceptable salt thereof may show an increase or decrease in the fluorescence intensity at an emission wavelength ranging from 430 nm to 680 nm.

An embodiment of the present invention may provide a composition for measuring antioxidant activity in living cells containing, as an active ingredient, the compound represented by Formula I, preferably the compound represented by any one of Formulas IV to VII, or a pharmaceutically acceptable salt thereof.

In the present invention, the measurement of the antioxidant activity may be measurement of the level of thiols in living cells, and measurement of the level of thiols may be measurement of the level of thiols in an organelle in a living cell, wherein the organelle may be the endoplasmic reticulum (ER).

In the present invention, as a result of measurement of the level of thiols, the compound in a free state may show a decrease in the fluorescence intensity at 550 to 680 nm as the level of thiols increases, and the compound in a thiol-bound state may show an increase in the fluorescence intensity at 430 to 550 nm as the level of thiols increases.

In the present invention, the fluorescence intensity may increase or decrease ratiometrically and reversibly.

In the present invention, measurement of the level of thiols may be performed by obtaining the ratio of the fluorescence intensity at 430 to 550 nm to the fluorescence intensity at 550 to 680 nm, and the ratio may be a relationship between the fluorescence intensity at 430 to 550 nm and the fluorescence intensity at 550 to 680 nm.

In the present invention, the relationship is a mathematical ratio between the fluorescence intensity at 430 to 550 nm and the fluorescence intensity at 550 to 680 nm, and the mathematical ratio may increase or decrease ratiometrically and reversibly depending on the amount of thiols in the endoplasmic reticulum (ER), thereby indicating the amount of thiols in intracellular organelles in real time.

In the present invention, measurement of the level of thiols may be quantitative or qualitative detection of thiols in the endoplasmic reticulum (ER), and measurement of the level of thiols may be real-time quantitative measurement.

In the present invention, measurement of the level of thiols may indicate the oxidative stress or degree of oxidation of the cell, and measurement of the level of thiols may indicate the degree of cellular senescence.

In the present invention, the thiols may be thiols present in glutathione (GSH), homocysteine (Hcy), cysteine (Cys) or protein cysteine residues.

When the composition containing the compound according to the present invention is used, it is possible to measure the antioxidant activity of the endoplasmic reticulum (ER), which is an intracellular organelle in all types of cells, including stem cells, thereby accurately measuring cell activity related to the antioxidant activity and screening highly active cells. The measurement of cellular activity by the use of the composition of the present invention includes, but is not limited to, measurement of antioxidant activity.

Another embodiment of the present invention may provide a composition for measuring the antioxidant activity of an intracellular organelle, the composition containing, as an active ingredient, the compound represented by Formula I, preferably the compound represented by any one of Formulas IV to VII, or a racemate, optical isomer, diastereomer, optical isomer mixture, or diastereomeric mixture thereof, or a pharmaceutically acceptable sale thereof.

Still another embodiment of the present invention may provide a method for screening a thiol enhancer or inhibitor in living cells, the method including steps of: (a) adding a composition containing the compound represented by Formula I, preferably the compound represented by any one of Formulas IV to VII, as an active ingredient, and a candidate substance simultaneously or sequentially in any order to living cells; (b) obtaining the ratio of the fluorescence intensity of the living cells at 430 to 550 nm to the fluorescence intensity at 550 to 680 nm and comparing the obtained ratio with standard data; (c) determining that the candidate substance is a thiol enhancer or inhibitor; and (d) determining that when the ratio of the fluorescence intensity at 430 to 550 nm to the fluorescence intensity at 550 to 680 nm decreases, the candidate substance is the thiol inhibitor, and when the ratio of the fluorescence intensity increases, the candidate substance is the thiol enhancer.

In the present invention, step (d) may comprise determining that, when the ratio (510/580 ratio) of the fluorescence intensity at 510 nm to the fluorescence intensity at 580 nm decreases, the candidate substance is the thiol inhibitor, and when the ratio (510/580 ratio) of the fluorescence intensity at 510 nm to the fluorescence intensity at 580 nm increases, the candidate substance is the thiol enhancer.

In the present invention, the ratio of the fluorescence intensity may be measured for the endoplasmic reticulum (ER).

Yet another embodiment of the present invention provides a kit for diagnosing oxidative stress-induced disease comprising the composition of the present invention. As used herein, the term "oxidative stress-induced disease" means a disease caused by oxidative stress, and has the same meaning as the term "relative oxygen species (ROS)-related disease".

In the present invention, the oxidative stress-induced disease may be aging, degenerative arthritis, cataract, Alzheimer's disease, cancer, fibrosis disease, diabetes, obesity, ischemia, ischemic reperfusion injury, inflammation, systemic lupus erythematosus, myocardial infarction, thrombotic stroke, hemorrhagic stroke, bleeding, spinal cord injury, Down syndrome, Crohn's disease, rheumatoid arthritis, uveitis, emphysema, gastric ulcer, oxygen toxicity, tumor, or radiation syndrome, without being limited thereto.

As used herein, the "kit" refers to a tool capable of evaluating the expression level of thiol by labeling the detectable compound of the present invention, which binds specifically to thiol, with a label. The labeling includes not only direct labeling of a detectable substance by reaction with a substrate, but also indirect labeling in which a color-generating label is conjugated by reactivity with another directly labeled reagent. The kit may include a chromogenic substrate solution to induce the chromogenic reaction with the label, a washing solution, and other solutions, and may be prepared to include reagent components used in the art. As the kit of the present invention, any kit known in the art may be used without limitation.

The kit of the present invention may further include one or more other constitutional compositions, solutions or devices suitable for the analysis method, and labels such as fluorescein and dye may be used, without being limited thereto.

Still yet another embodiment of the present invention provides a method for measuring antioxidant activity in living cells, the method including steps of: (a) measuring in real time the ratio of the fluorescence intensity at 430 to 550 nm to the fluorescence intensity at 550 to 680 nm in living cells; (b) adding the composition of the present invention to the living cells; (c) adding an oxidizing agent to the cells of step (b); and (d) observing a change in the ratio of the fluorescence intensities.

In the present invention, step (a) may comprise measuring in real time the ratio (510/580 ratio) of the fluorescence intensity at 510 nm to the fluorescence intensity at 580 nm.

In the present invention, the method for measuring antioxidant activity may further comprise, after step (d), a step of measuring the time for the ratio of the fluorescence intensities to return to either the fluorescence intensity ratio of the living cells to which the oxidizing agent was not added or the fluorescence intensity ratio shown before the oxidizing agent is added, wherein it may be determined that the shorter the time, the higher the antioxidant activity.

In the present invention, the method for measuring antioxidant activity may further comprise, after step (d), a step of measuring the integrated value of the difference between the fluorescence intensity ratio of the living cells to which the oxidizing agent was not added and the fluorescence intensity ratio of the living cells to which the oxidizing agent was added, from a time point at which the oxidizing agent was added to a time point at which the fluorescence intensity ratio returns to the fluorescence intensity ratio shown before the oxidizing agent is added, wherein it may be determined that the smaller the integrated value, the higher the antioxidant activity.

In the present invention, the method for measuring antioxidant activity may further comprise, after step (d), a step of determining the minimum concentration of the oxidizing agent, at which the fluorescence intensity ratio of the living cells to which the oxidizing agent was added starts to decrease, wherein it may be determined that the higher the minimum concentration, the higher the antioxidant activity.

In the present invention, the measuring method may be performed for the endoplasmic reticulum (ER), which is an organelle in living cells.

### Advantageous Effects

Using the composition comprising the compound according to the present invention, it is possible to measure the antioxidant activity of the endoplasmic reticulum (ER), which is an organelle in living cells, particularly stem cells, and it is possible to screen highly active stem cells based on the result of measuring the antioxidant activity of the endoplasmic reticulum (ER).

### Brief Description of Drawings

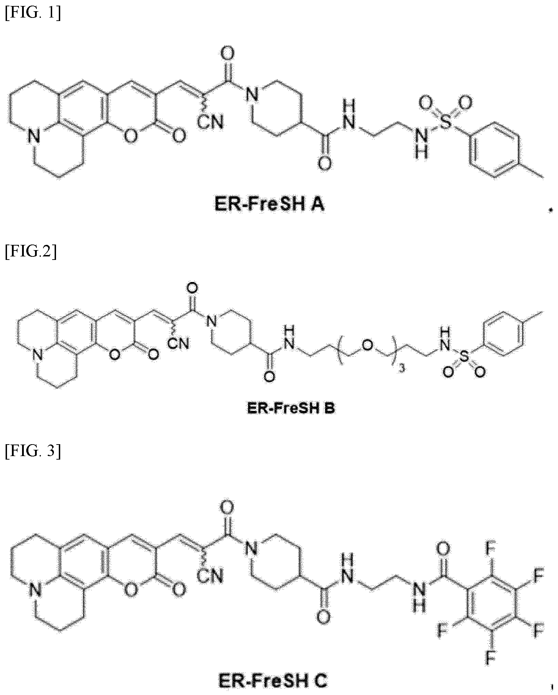

FIG. 1 shows the structure of ER-FT represented by Formula IV.
FIG. 2 shows the structure of ER-FT represented by Formula V.
FIG. 3 shows the structure of ER-FT represented by Formula VI.
FIG. 4 shows the structure of ER-FT represented by Formula VII.
FIG. 5 depicts confocal microscopic images showing the results of observing of ER-FTs represented by Formulas IV to VII in the endoplasmic reticulum of UC-MSCs.
FIG. 6 shows toxicity test results indicating the IC₅₀ values of ER-FTs represented by Formulas IV to VII.
FIG. 7 shows the experimental results of measuring the intensity at a 510 nm wavelength, the intensity at a 580 nm wavelength, and the ratio of the intensities at the two wavelengths as a function of the retention time of ER-FTs represented by Formulas IV to VI in UC-MSCs.
FIG. 8 shows the experimental results of measuring the intensity at a 510 nm wavelength, the intensity at a 580 nm wavelength, and the ratio of the intensities at the two wavelengths as a function of the retention time of ER-FTs represented by Formulas IV, V and VI in UC-MSCs.
FIG. 9 shows confocal microscope images of UC-MSCs to which diamide was added after treatment with the ER-FT represented by Formula IV.
FIG. 10 shows confocal microscope images of UC-MSCs to which diamide and DTT were added after treatment with the ER-FT represented by Formula IV.
FIG. 11 shows confocal microscope images of the endoplasmic reticulum of UC-MSCs to which diamide was added after treatment with the ER-FT represented by Formula V.
FIG. 12 shows confocal microscope images of the endoplasmic reticulum of UC-MSCs to which diamide and DTT were added after treatment with the ER-FT represented by Formula V.
FIG. 13 shows confocal microscope images of the endoplasmic reticulum of UC-MSCs to which diamide was added after treatment with the ER-FT represented by Formula VI.
FIG. 14 shows confocal microscope images of the endoplasmic reticulum of UC-MSCs to which diamide and DTT were added after treatment with the ER-FT represented by Formula VI.
FIG. 15 shows confocal microscope images of the endoplasmic reticulum of UC-MSCs to which diamide was added after treatment with the ER-FT represented by Formula VII.
FIG. 16 shows confocal microscope images of the endoplasmic reticulum of UC-MSCs to which diamide and DTT were added after treatment with the ER-FT represented by Formula VII.

### Best Mode

An object of the present invention is to provide endoplasmic reticulum fluorescent real-time SH group-tracers (ER-FTs) represented by Formulas IV to VII.

### Mode for Invention

Hereinafter, the present invention will be described in more detail with reference to examples. These examples are only for illustrating the present invention in more detail, and it will be apparent to those of ordinary skill in the art that the scope of the present invention according to the subject matter of the present invention is not limited by these examples.

### [Preparation Example] Synthesis of compounds for measuring antioxidant activity of endoplasmic reticulum (ER)

Methods for preparing compounds (ER-FTs) used to measure the antioxidant activity of endoplasmic reticulum (ER) are as follows.

### 1. Method for preparing ER-FresH A (Formula IV)

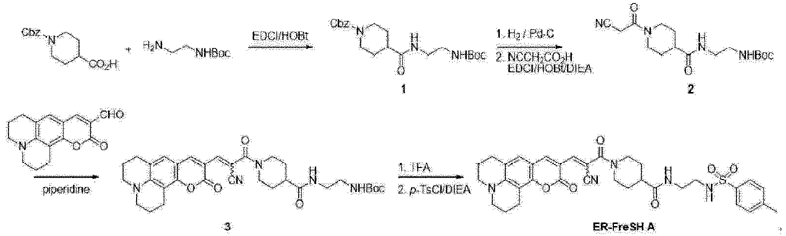

**Compound 1**

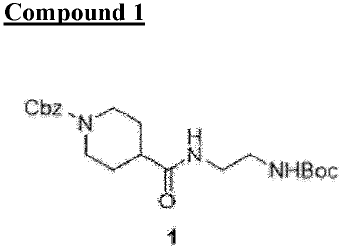

1-(carbobenzyloxy)-4-piperidinecarboxylic acid (0.30 g, 1.1 mmol), N-(tert-butoxycarbonyl)-ethylenediamine (0.19 mL, 1.0 equivalent), 1-hydroxybenzotriazol (HOBt; 0.23 g, 1.5 equivalents), and 1-ethyl-3-(3-dimethylaminopropyl)-carbodiimide (EDCI; 0.29 g, 1.5 equivalents) were dissolved in 5 mL of N,N-dimethylformamide (DMF), and the solution was stirred at room temperature for 15 hours. The resulting mixture was diluted with EtOAc and then washed with an aqueous solution of NaHCO₃ and a saturated aqueous solution of NaCl. The organic layer was separated, dried with Na₂SO₄, and then filtered. The filtrate was distilled under reduced pressure to remove the solvent. The residue was purified by SiO₂ column chromatography to obtain compound 1 (0.45 g, 98%).

¹H NMR (500 MHz, CDCl₃): δ (ppm) = 7.31-7.37 (m, 5H), 6.63 (br, 1H), 5.12-5.14 (m, 3H), 4.18-4.21 (m, 2H), 3.32-3.35 (m, 2H), 3.25-3.28 (m, 2H), 2.80 to 2.85 (m, 2H), 2.22-2.27 (m, 1H), 1.80 to 1.84 (m, 2H), 1.60 to 1.67 (m, 2H), 1.42 (s, 9H).

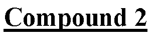

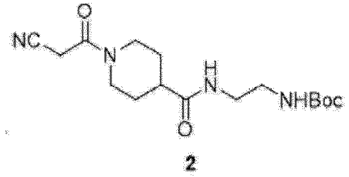

Palladium on activated carbon (Pd-C; 10 wt%, 45 mg) was added to a solution of compound 1 (0.45 g, 1.1 mmol) in 10 mL of MeOH, followed by stirring under H₂ gas (1 atm) for 15 hours. The mixture was filtered through a celite layer, and the filtrate was distilled under reduced pressure to remove the solvent. A portion (0.15 g, 0.55 mmol) of the solid obtained by vacuum drying, cyanoacetic acid (48 mg, 1.0 equivalent), HOBt (0.11 g, 1.3 equivalents), EDCI (0.14 g, 1.3 equivalents), and N,N-diisopropylethylamine (DIEA; 0.15 mL, 1.5 equivalents) were dissolved in 5 mL of DMF, and the solution was stirred at room temperature for 14 hours. The solvent was removed by distillation under reduced pressure, and the residue was purified by SiO₂ column chromatography to obtain compound 2 (0.17 g, 90%).

¹H NMR (500 MHz, CDCl₃): δ (ppm) = 6.62 (br, 1H), 4.96 (br, 1H), 4.46-4.49 (m, 1H), 3.74-3.77 (m, 1H), 3.51 (s, 2H), 3.34-3.37 (m, 2H), 3.28-3.31 (m, 2H), 3.18-3.24 (m, 1H), 2.80-2.85 (m, 1H), 2.34-2.40 (m, 1H), 1.90-1.98 (m, 2H), 1.63-1.80 (m, 2H), 1.44 (s, 9H).

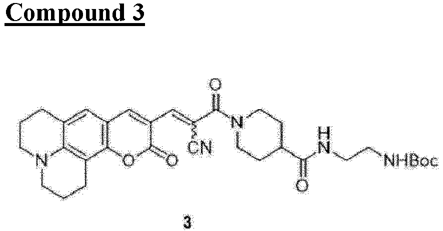

10-oxo-2,3,5,6-tetrahydro-1H,4H,10H-11-oxa-3a-azabenzo[de]anthracene-9-carbaldehyde (0.12 g, 0.45 mmol), compound 2 (0.17 g, 1.1 equivalents), and piperidine (44 µL, 1.0 equivalent) were dissolved in 3 mL of 2-propanol, and the solution was heated at 60°C for 16 hours and then cooled to room temperature. The solvent was removed by distillation under reduced pressure, and the residue was purified by SiO₂ column chromatography to obtain compound 3 (0.25 g, 96%).

¹H NMR (500 MHz, CDCl₃): δ (ppm) = ((E)-conformer) 8.63 (s, 1H), 7.94 (s, 1H), 6.99 (s, 1H), 6.55 (br, 1H), 4.97 (br, 1H), 4.29 (br, 2H), 3.28-3.38 (m, 8H), 3.06 (br, 2H), 2.86 (t, J = 6.3 Hz, 2H), 2.76 (t, J = 6.2 Hz, 2H), 2.36-2.42 (m, 1H), 1.94-2.00 (m, 6H), 1.71-1.80 (m, 2H), 1.44 (s, 9H).

### ER-FresH A

Compound 3 (0.25 g, 0.42 mmol) was dissolved in a mixed solution of trifluoroacetic acid (TFA; 2 mL)/CH₂Cl₂ (2 mL), followed by stirring at room temperature for 2 hours. After the solvent was removed by distillation under reduced pressure, the remaining compound, p-toluenesulfonyl chloride (TsCl; 32 mg, 1.0 equivalent), and DIEA (58 µL, 2.0 equivalents) were dissolved in 2 mL of DMF, and the solution was stirred at room temperature for 4 hours. The solvent was removed by distillation under reduced pressure, and the residue was purified by SiO₂ column chromatography to obtain ER-FReSH A (23 mg, 22%).

¹H NMR (500 MHz, CDCl₃): δ (ppm) = ((E)-conformer) 8.63 (s, 1H), 7.92 (s, 1H), 7.72 (d, J = 8.0 Hz, 2H), 7.31 (d, J = 8.0 Hz, 2H), 7.00 (s, 1H), 6.52 (t, J = 5.9 Hz, 1H), 5.66 (t, J = 6.1 Hz, 1H), 4.26 (br, 2H), 3.32-3.38 (m, 6H), 3.02-3.08 (m, 4H), 2.85 (t, J = 6.4 Hz, 2H), 2.76 (t, J = 6.3 Hz, 2H), 2.41 (s, 3H), 2.36-2.42 (m, 1H), 1.86-1.99 (m, 6H), 1.68-1.77 (m, 2H).

### 2. Method for preparing ER-FresH B (Formula V)

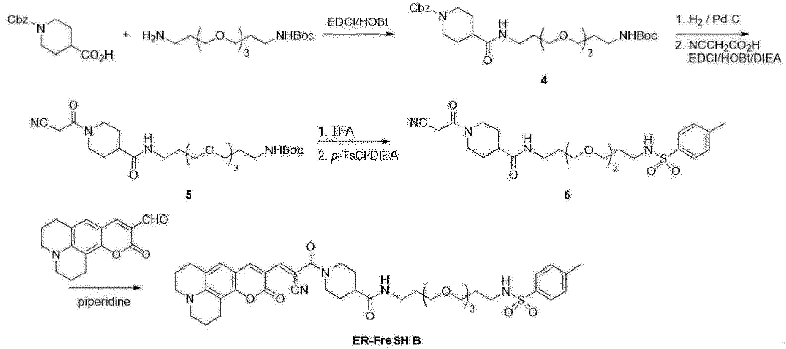

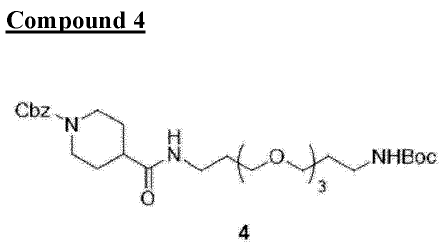

1-(carbobenzyloxy)-4-piperidinecarboxylic acid (0.24 g, 0.87 mmol), N-(tert-butoxycarbonyl)-4,7,10 to trioxa-1,13-tridecanediamine (0.28 g, 1.0 equivalent), HOBt (0.18 g, 1.3 equivalents), and EDCI (0.22 g, 1.3 equivalents) were dissolved in 10 mL of DMF, and the solution was stirred at room temperature for 18 hours. The resulting mixture was diluted with EtOAc and then washed with an aqueous solution of NaHCO₃ and a saturated aqueous solution of NaCl. The organic layer was separated, dried with Na₂SO₄ and then filtered, and the filtrate was distilled under reduced pressure to remove the solvent. The residue was purified by SiO₂ column chromatography to obtain compound 4 (0.49 g, 99%).

¹H NMR (500 MHz, CDCl₃): δ (ppm) = 7.31-7.36 (m, 5H), 6.37 (br, 1H), 5.12 (s, 2H), 4.96 (br, 1H), 4.17-4.23 (m, 2H), 3.57-3.64 (m, 10H), 3.52 (t, J = 5.9 Hz, 2H), 3.35-3.39 (m, 2H), 3.19-3.23 (m, 2H), 2.81-2.86 (m, 2H), 2.20-2.26 (m, 1H), 1.73-1.83 (m, 6H), 1.61-1.68 (m, 2H), 1.43 (s, 9H).

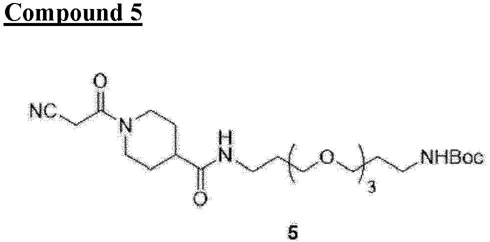

Pd-C (10 wt%, 49 mg) was added to a solution of compound 4 (0.49 g, 0.87 mmol) in 5 mL of MeOH, followed by stirring under H₂ gas (1 atm) for 14 hours. The mixture was filtered through a celite layer, and the filtrate was distilled under reduced pressure to remove the solvent. The solid obtained by vacuum drying, cyanoacetic acid (74 mg, 1.0 equivalent), HOBt (0.17 g, 1.3 equivalents), EDCI (0.22 g, 1.3 equivalents), and DIEA (0.23 mL, 1.5 equivalents) were dissolved in 5 mL of DMF, and the solution was stirred at room temperature for 12 hours. The solvent was removed by distillation under reduced pressure, and the residue was purified by SiO₂ column chromatography to obtain compound 5 (0.34 g, 79%).

¹H NMR (500 MHz, CDCl₃): δ (ppm) = 6.53 (br, 1H), 4.95 (br, 1H), 4.43-4.47 (m, 1H), 3.75-3.79 (m, 1H), 3.57-3.66 (m, 10H), 3.53 (t, J = 6.0 Hz, 2H), 3.52 (s, 2H), 3.36-3.39 (m, 2H), 3.18-3.23 (m, 3H), 2.81-2.87 (m, 1H), 2.33-2.39 (m, 1H), 1.87-1.94 (m, 2H), 1.73-1.82 (m, 5H), 1.64-1.72 (m, 1H), 1.44 (s, 9H).

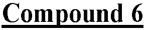

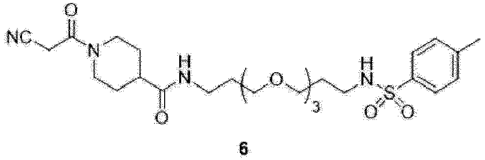

Compound 5 (0.13 g, 0.26 mmol) was dissolved in a mixed solution of TFA (2 mL)/CH₂Cl₂ (1 mL), followed by stirring at room temperature for 3 hours. After the solvent was removed by distillation under reduced pressure, the remaining compound and DIEA (0.12 mL, 2.6 equivalents) were dissolved in 3 mL of CH₂Cl₂, and the solution was cooled to 0°C. p-TsCl (63 mg, 1.3 equivalents) was added thereto, followed by stirring for 4 hours while warming to room temperature. The mixture was diluted with CH₂Cl₂ and then washed with a saturated aqueous solution of NaCl. The organic layer was separated, dried with Na₂SO₄, and then filtered, and the filtrate was distilled under reduced pressure to remove the solvent. The residue was purified by SiO₂ column chromatography to obtain compound 6 (68 mg, 47%).

¹H NMR (500 MHz, CDCl₃): δ (ppm) = 7.77 (d, J = 8.1 Hz, 2H), 7.32 (d, J = 8.1 Hz, 2H), 6.62 (t, J = 5.2 Hz, 1H), 5.85 (t, J = 5.6 Hz, 1H), 4.42-4.46 (m, 1H), 3.67-3.73 (m, 5H), 3.62-3.64 (m, 2H), 3.58 (t, J = 5.8 Hz, 2H), 3.50-3.55 (m, 6H), 3.33-3.37 (m, 2H), 3.14-3.20 (m, 1H), 3.04-3.08 (m, 2H), 2.74-2.80 (m, 1H), 2.43 (s, 3H), 2.35-2.41 (m, 1H), 1.82-1.91 (m, 2H), 1.58-1.80 (m, 6H).

### ER-FresH B

10-oxo-2,3,5,6-tetrahydro-1H,4H,10H-11-oxa-3a-azabenzo[de]anthracene-9-carbaldehyde (30 mg, 0.11 mmol), compound 6 (68 mg, 1.1 equivalents), and piperidine (11 µL, 1.0 equivalent) were dissolved in 1 mL of 2-propanol, and the solution was stirred at room temperature for 15 hours. The solvent was removed by distillation under reduced pressure, and the residue was purified by SiO₂ column chromatography to obtain ER-FT B (77 mg, 86%).

¹H NMR (500 MHz, CDCl₃): δ (ppm) = ((E)-conformer) 8.63 (s, 1H), 7.92 (s, 1H), 7.77 (d, J = 8.2 Hz, 2H), 7.30 (d, J = 8.2 Hz, 2H), 6.99 (s, 1H), 6.53 (t, J = 5.3 Hz, 1H), 5.74 (t, J = 5.7 Hz, 1H), 4.25 (br, 2H), 3.67-3.69 (m, 4H), 3.62-3.64 (m, 2H), 3.53-3.60 (m, 4H), 3.50 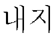 3.52 (m, 2H), 3.32-3.39 (m, 6H), 3.04-3.08 (m, 2H), 3.02 (br, 2H), 2.87 (t, J = 6.5 Hz, 2H), 2.76 (t, J = 6.0 Hz, 2H), 2.42 (s, 3H), 2.34-2.41 (m, 1H), 1.95-2.00 (m, 4H), 1.89-1.92 (m, 2H), 1.66-1.81 (m, 6H).

### 3. Synthesis of ER-FresH C (Formula VI)

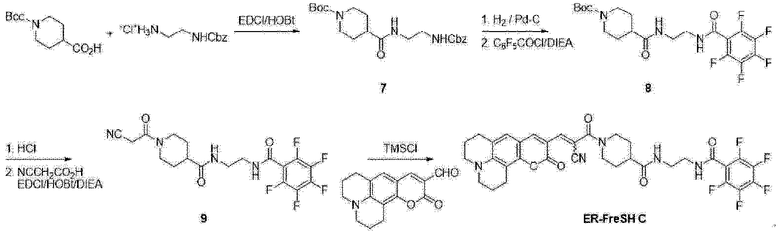

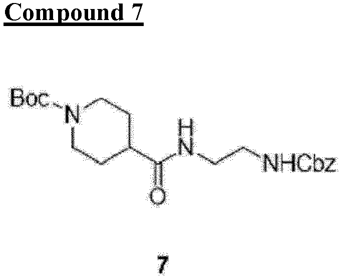

1-(tert-butoxycarbonyl)-4-piperidinecarboxylic acid (0.30 g, 1.3 mmol), N-carbobenzyloxy-ethylenediamine hydrochloride (0.31 g, 1.0 equivalent), HOBt (0.27 g, 1.3 equivalents), EDCI (0.33 g, 1.3 equivalents), and DIEA (0.34 mL, 1.5 equivalents) were dissolved in 10 mL of DMF, and the solution was stirred at room temperature for 18 hours. The resulting mixture was diluted with EtOAc and then washed with a saturated aqueous solution of NaCl. The organic layer was separated, dried with Na₂SO₄, and then filtered, and the filtrate was distilled under reduced pressure to remove the solvent. The residue was purified by SiO₂ column chromatography to obtain compound 7 (0.53 g, 100%).

¹H NMR (500 MHz, CDCl₃): δ (ppm) = 7.31-7.38 (m, 5H), 6.24 (br, 1H), 5.22 (br, 1H), 5.10 (s, 2H), 4.08-4.14 (m, 2H), 3.33-3.39 (m, 4H), 2.68-2.73 (m, 2H), 2.14-2.20 (m, 1H), 1.73-1.76 (m, 2H), 1.52-1.60 (m, 2H), 1.46 (s, 9H).

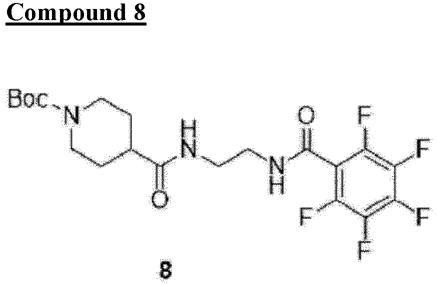

Pd-C (10 wt%, 53 mg) was added to a solution of compound 7 (0.53 g, 1.3 mmol) in 10 mL of MeOH, followed by stirring under H₂ gas (1 atm) for 2 hours. The mixture was filtered through a celite layer, and the filtrate was distilled under reduced pressure to remove the solvent. A portion (0.10 g, 0.37 mmol) of the solid obtained by vacuum drying and DIEA (0.13 mL, 2.0 equivalents) were dissolved in 3 mL of CH₂Cl₂, and pentafluorobenzoyl chloride (52 µL, 1.0 equivalent) was added to the solution, followed by stirring for 2 hours. The mixture was diluted with CH₂Cl₂ and then washed with an aqueous solution of NaHCO₃ and a saturated aqueous solution of NaCl. The organic layer was separated, dried with Na₂SO₄, and then filtered, and the filtrate was distilled under reduced pressure to remove the solvent. The residue was purified by SiO₂ column chromatography to obtain compound 8 (0.12 g, 73%).

¹H NMR (500 MHz, CDCl₃): δ (ppm) = 7.33 (br, 1H), 6.28 (t, J = 5.3 Hz, 1H), 4.10 to 4.15 (m, 2H), 3.58-3.61 (m, 2H), 3.49-3.53 (m, 2H), 2.70 to 2.75 (m, 2H), 2.25 (tt, J = 11.6 Hz, J = 3.7 Hz, 1H), 1.77-1.80 (m, 2H), 1.52-1.61 (m, 2H), 1.45 (s, 9H). ¹⁹F NMR (470 MHz, CDCl₃): δ (ppm) = -140.8 (2F), -150.6 (1F), -159.9 (2F).

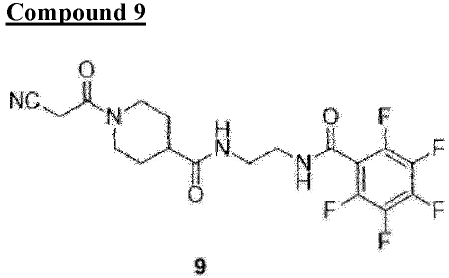

Compound 8 (0.12 g, 0.27 mmol) was dissolved in 2 mL of HCl solution (4 M, dioxane), followed by stirring at room temperature for 2 hours. After the solvent was removed by distillation under reduced pressure, the remaining compound, cyanoacetic acid (24 mg, 1.0 equivalent), HOBt (56 mg, 1.3 equivalents), EDCI (70 mg, 1.3 equivalents), and DIEA (73 µL, 1.5 equivalents) were dissolved in 2 mL of DMF, and the solution was stirred at room temperature for 17 hours. The mixture was diluted with CH₂Cl₂ and then washed with a saturated aqueous solution of NaCl. The organic layer was separated, dried with Na₂SO₄, and then filtered, and the filtrate was distilled under reduced pressure to remove the solvent. The residue was purified by SiO₂ column chromatography to obtain compound 9 (44 mg, 37%).

¹H NMR (500 MHz, DMSO-d6): δ (ppm) = 8.94 (t, J = 5.7 Hz, 1H), 7.91 (t, J = 5.6 Hz, 1H), 4.24-4.28 (m, 1H), 3.97-4.09 (m, 2H), 3.63-3.67 (m, 1H), 3.30-3.34 (m, 2H), 3.18-3.21 (m, 2H), 3.00-3.05 (m, 1H), 2.64-2.69 (m, 1H), 2.31-2.38 (m, 1H), 1.68-1.72 (m, 2H), 1.51-1.59 (m, 1H), 1.34-1.43 (m, 1H). ¹⁹F NMR (470 MHz, DMSO-d6): δ (ppm) = -141.9 (2F), -153.0 (1F), -161.4 (2F).

### ER-FresH C

Compound 9 (44 mg, 0.10 mmol) and 10-oxo-2,3,5,6-tetrahydro-1H,4H,10H-11-oxa-3a-azabenzo[de]-anthracene-9-carbaldehyde (30 mg, 1.1 equivalents) were dissolved in 1 mL of DMF, and chlorotrimethylsilane (TMSC1; 39 µL, 3.0 equivalents) was added to the solution, followed by stirring at 130°C for 5 hours. The mixture was cooled to room temperature, diluted with CH₂Cl₂, and then washed with a saturated aqueous solution of NaCl. The organic layer was separated, dried with Na₂SO₄, and then filtered, and the filtrate was distilled under reduced pressure to remove the solvent. The residue was purified by SiO₂ column chromatography to obtain ER-FT C (33 mg, 48%).

¹H NMR (500 MHz, CDCl₃): δ (ppm) = ((E)-conformer) 8.61 (s, 1H), 7.89 (s, 1H), 7.48 (br, 1H), 6.99 (s, 1H), 6.62 (br, 1H), 4.29 (br, 2H), 3.97-4.09 (m, 2H), 3.57-3.61 (m, 2H), 3.48-3.52 (m, 2H), 3.33-3.39 (m, 4H), 3.03 (br, 2H), 2.73-2.86 (m, 4H), 2.40-2.47 (m, 1H), 1.89-2.04 (m, 6H), 1.72-1.80 (m, 2H). ¹⁹F NMR (470 MHz, CDCl₃): δ (ppm) = -140.7 (2F), -150.8 (1F), -159.9 (2F).

### Synthesis of ER-FresH D (Formula VII)

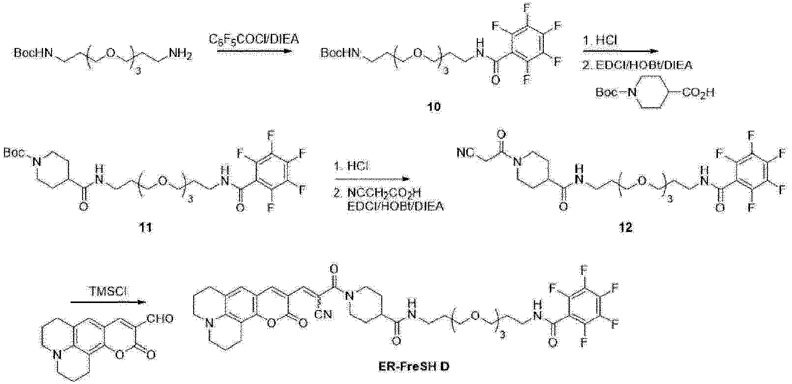

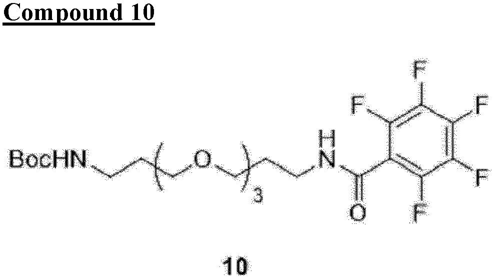

N-(tert-butoxycarbonyl)-4,7,10 to trioxa-1,13-tridecanediamine (0.10 g, 0.31 mmol) and DIEA (0.1 mL, 2.0 equivalents) were dissolved in 3 mL of CH₂Cl₂, and pentafluorobenzoyl chloride (44 µL, 1.0 equivalent) was added to the solution, followed by stirring for 2 hours. The mixture was diluted with CH₂Cl₂ and then washed with an aqueous solution of NaHCO₃ and a saturated aqueous solution of NaCl. The organic solvent was separated, dried with Na₂SO₄, and then filtered, and the filtrate was distilled under reduced pressure to remove the solvent. The residue was purified by SiO₂ column chromatography to obtain compound 10 (0.15 g, 94%).

¹H NMR (500 MHz, CDCl₃): δ (ppm) = 7.18 (br, 1H), 4.88 (br, 1H), 3.65 (t, J = 5.8 Hz, 2H), 3.58-3.61 (m, 6H), 3.46-3.52 (m, 6H), 3.17-3.21 (m, 2H), 1.88-1.92 (m, 2H), 1.70 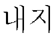 1.75 (m, 2H), 1.41 (s, 9H). ¹⁹F NMR (470 MHz, CDCl₃): δ (ppm) = -140.7 (2F), -151.8 (1F), -160.6 (2F).

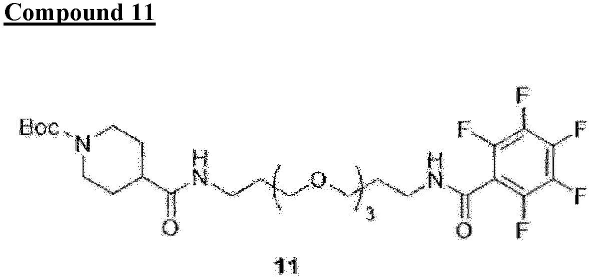

Compound 10 (0.15 g, 0.29 mmol) was dissolved in 2 mL of HCl solution (4 M, dioxane), followed by stirring at room temperature for 2 hours. After the solvent was removed by distillation under reduced pressure, the remaining compound, 1-(tert-butoxycarbonyl)-4-piperidinecarboxylic acid (69 mg, 1.0 equivalent), HOBt (59 mg, 1.3 equivalents), EDCI (73 mg, 1.3 equivalents), and DIEA (76 µL, 1.5 equivalents) were dissolved in 3 mL of DMF, and the solution was stirred at room temperature for 16 hours. The resulting mixture was diluted with EtOAc and then washed with an aqueous solution of NaHCO₃ and a saturated aqueous solution of NaCl. The organic layer was separated, dried with Na₂SO₄, and then filtered, and the filtrate was distilled under reduced pressure to remove the solvent. The residue was purified by SiO₂ column chromatography to obtain compound 11 (0.17 g, 93%).

¹H NMR (500 MHz, CDCl₃): δ (ppm) = 7.31 (br, 1H), 6.24 (br, 1H), 4.07-4.12 (m, 2H), 3.53-3.63 (m, 12H), 3.49-3.52 (m, 2H), 3.33-3.37 (m, 2H), 2.70-2.75 (m, 2H), 2.18 (tt, J = 11.6 Hz, J = 3.6 Hz, 1H), 1.88-1.92 (m, 2H), 1.73-1.81 (m, 4H), 1.52-1.60(m, 2H), 1.43 (s, 9H). ¹⁹F NMR (470 MHz, CDCl₃): δ (ppm) = -140.7 (2F), -151.8 (1F), -160.6 (2F).

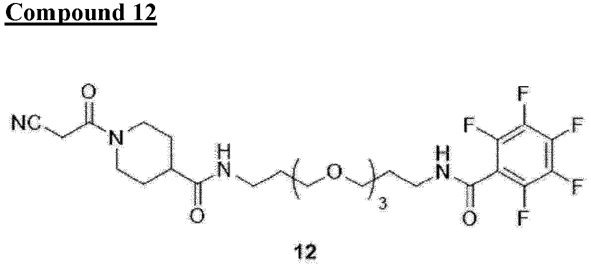

Compound 11 (0.17 g, 0.27 mmol) was dissolved in 2 mL of HCl solution (4 M, dioxane), followed by stirring at room temperature for 1 hour. After the solvent was removed by distillation under reduced pressure, the remaining compound, cyanoacetic acid (23 mg, 1.0 equivalent), HOBt (54 mg, 1.3 equivalents), EDCI (67 mg, 1.3 equivalents), and DIEA (70 µL, 1.5 equivalents) were dissolved in 2 mL of DMF, and the solution was stirred at room temperature for 18 hours. The resulting mixture was diluted with CH₂Cl₂ and then washed with an aqueous solution of NaHCO₃ and a saturated aqueous solution of NaCl. The organic layer was separated, dried with Na₂SO4, and then filtered, and the filtrate was distilled under reduced pressure to remove the solvent. The residue was purified by SiO₂ column chromatography to obtain compound 12 (0.11 g, 69%).

¹H NMR (500 MHz, CDCl₃): δ (ppm) = 7.16 (br, 1H), 6.39 (br, 1H), 4.41-4.46 (m, 1H), 3.73-3.77 (m, 1H), 3.53-3.63 (m, 12H), 3.50-3.52 (m, 4H), 3.33-3.37 (m, 2H), 3.17-3.23 (m, 1H), 2.81-2.86 (m, 1H), 2.35 (tt, J = 10.8 Hz, J = 4.0 Hz, 1H), 1.87-1.96 (m, 4H), 1.71-1.78 (m, 3H), 1.60 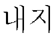 1.68 (m, 1H). ¹⁹F NMR (470 MHz, CDCl₃): δ (ppm) = -140.7 (2F), -151.6 (1F), -160.5 (2F).

### ER-FresH D

Compound 12 (57 mg, 96 µmol) and 10-oxo-2,3,5,6-tetrahydro-1H,4H, 10H-11-oxa-3a-azabenzo[de]-anthracene-9-carbaldehyde (28 mg, 1.1 equivalents) were dissolved in 1 mL of DMF, and TMSCl (16 µL, 1.3 equivalents) was added to the solution, followed by stirring at 130°C for 14 hours. The resulting mixture was cooled to room temperature, diluted with CH₂Cl₂, and then washed with a saturated aqueous solution of NaCl. The organic layer was separated, dried with Na₂SO₄, and then filtered, and the filtrate was distilled under reduced pressure to remove the solvent. The residue was purified by SiO₂ column chromatography to obtain ER-FT D (31 mg, 38%).

¹H NMR (500 MHz, CDCl₃): δ (ppm) = ((E)-conformer) 8.61 (s, 1H), 7.88 (s, 1H), 7.35 (br, 1H), 6.99 (s, 1H), 6.44 (br, 1H), 4.26 (br, 2H), 3.51-3.63 (m, 14H), 3.33-3.39 (m, 6H), 3.07 (br, 2H), 2.82-2.87 (m, 2H), 2.73-2.78 (m, 2H), 2.31-2.40 (m, 1H), 1.87-2.04 (m, 8H), 1.68-1.79 (m, 4H). ¹⁹F NMR (470 MHz, CDCl₃): δ (ppm) = -140.6 (2F), -151.8 (1F), -160.5 (2F).

The ER-FresH A/B/C/D synthesized in the Preparation Example are classified by R₃ as shown in Table 1 below.

**[Table 1]**

| R₃ | Compound |
|---|---|
| -(CH₂)ₘ-R₄ | Formula IV (ER-FresH A) |
| -(CH₂)ₙ-(CH₂OCH₂)ₚ-(CH₂)_{q}-R₄ | Formula V (ER-FresH B) |
| -(CH₂)ₘ-R₅ | Formula VI (ER-FresH C) |
| -(CH₂)ₙ-(CH₂OCH₂)ₚ-(CH₂)_{q}-R₅ | Formula VII (ER-FresH D) |

### [Experimental Example] Experimental Methods

### 1. In vitro reaction of endoplasmic reticulum fluorescent real-time SH group-tracer (ER-FT) derivative compound (hereinafter referred to as any one of Formulas IV to VII) with thiol compound

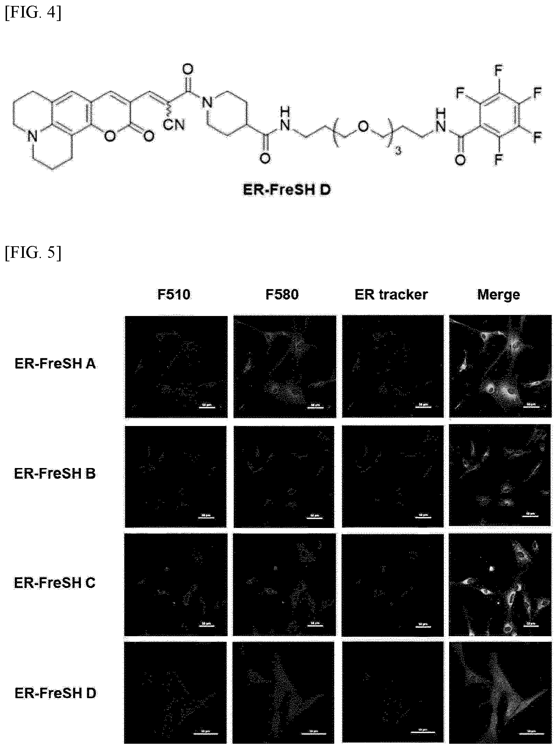

FIG. 5 depicts confocal microscope images showing the results of observing ER-FTs represented by Formulas IV to VII in the endoplasmic reticulum of UC-MSCs.

(1) Umbilical cord mesenchymal stem cells (UC-MSCs) and HeLa cells were seeded into confocal dishes (UC-MSC: 5 × 10⁴, HeLa cells: 2 × 10⁵) and then cultured for 24 hours. (2) 2 ml of each of ER-FTs (Formulas IV to VII) was prepared for a medium for each cell type at a concentration of 10 to 20 µM. (3) Each ER-FT was added to each dish, followed by staining and then incubation in an incubator at 37°C for 30 minutes. (4) 2 µ1 of 1 mM ER tracker Red was added to each dish and mixed well, followed by incubation for 30 minutes. (5) After medium suction, 2 ml of HBSS was added.

The cells were imaged with a confocal microscope, and the results are shown in FIG. 5.

### 2. Cytotoxicity test

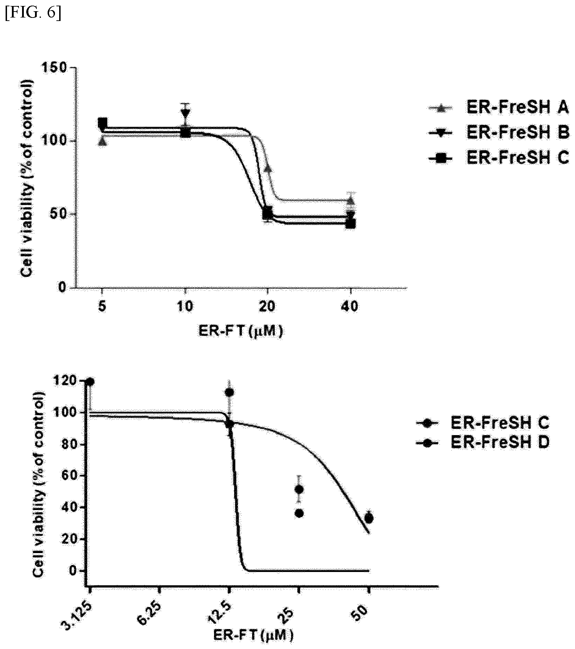

FIG. 6 shows toxicity test results indicating the IC₅₀ values of ER-FTs represented by Formulas IV to VII.

(1) UC-MSCs (4 × 10³ cells/well) were cultured in 96-well dishes for 24 hours. (2) The cells were treated with 2 ml of each of ER-FT derivative compounds (Formulas IV to VII) at concentrations of 5, 10, 20 and 40 µM and then incubated at 37°C for 24 hours. (3) 10 µl of 10X EZ-cytox was added to each well, followed by incubation for 3 hours. (4) The absorbance at 450 nm was measured using a plate reader (reference wavelength: 600 to 650 nm).

As a result of the cytotoxicity test, as shown in FIG. 6 and Table 2 below, it was confirmed that Formula IV (ER-FreSH A) had the lowest toxicity.

**[Table 2]**

| | Formula IV | Formula V | Formula VI | Formula VII |
|---|---|---|---|---|
| IC₅₀ (νM) | 43.4 | 36.39 | 33.44 | 13.28 |

### 3. Measurement of intracellular retention time of each ER-FT derivative compound

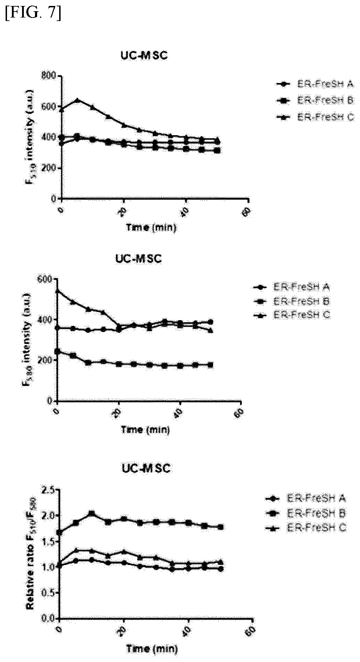

FIG. 7 shows the experimental results of measuring the intensity at a 510 nm wavelength, the intensity at a 580 nm wavelength, and the ratio of the intensities at the two wavelengths as a function of the retention time of ER-FTs represented by Formulas IV to VI in UC-MSCs.

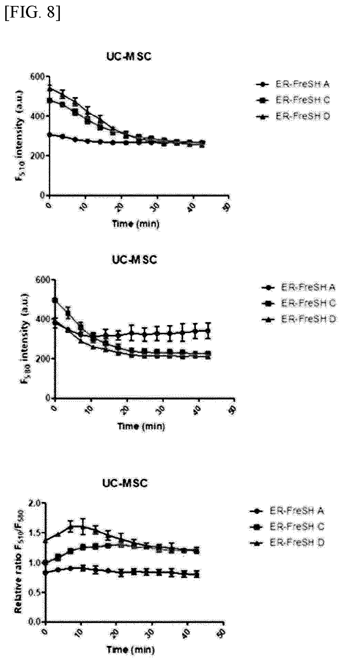

FIG. 8 shows the experimental results of measuring the intensity at a 510 nm wavelength, the intensity at a 580 nm wavelength, and the ratio of the intensities at the two wavelengths as a function of the retention time of ER-FTs represented by Formulas IV, VI and VII in UC-MSCs.

(1) UC-MSCs were seeded into 96-well plates at a density of 4,000 cells/well and then cultured 24 hours. (2) Cell media containing each of ER-FTs (Formulas IV to VI) at concentrations of 10, 15 and 20 µM were prepared. (3) After medium suction, ER-FT staining was performed, followed by incubation in an incubator at 37°C for 1 hour. (4) After medium suction, 100 µl of HBSS was added to each well.

As a result of Operetta imaging, as shown in Figures 7 and 8, Formulas IV and V showed constant fluorescence intensity in UC-MSCs. The fluorescence intensities of Formulas VI and VII were high at the initial stage, but decreased gradually during the initial 20 minutes, and then were similar to that of the other ER-FT.

As shown in FIGS. 7 and 8, taking all the results together, Formulas VI and VII remained in the cells for a long time, and the time to wash was required. In addition, after they were completely washed, they showed similar fluorescence intensities.

### 4. Measurement of reactivity of each ER-FT derivative compound

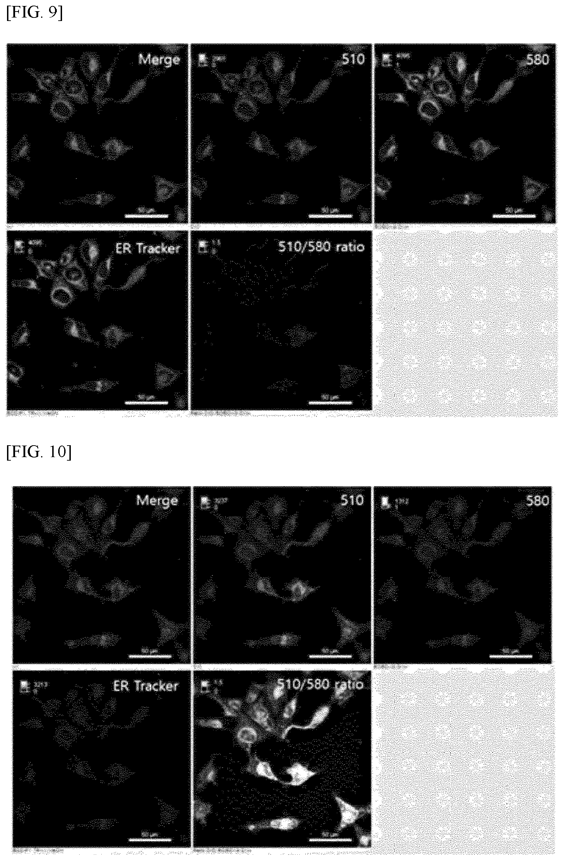

FIG. 9 shows confocal microscope images of UC-MSCs to which diamide was added after treatment with the ER-FT represented by Formula IV.

FIG. 10 shows confocal microscope images of UC-MSCs to which diamide and DTT were added after treatment with the ER-FT represented by Formula IV.

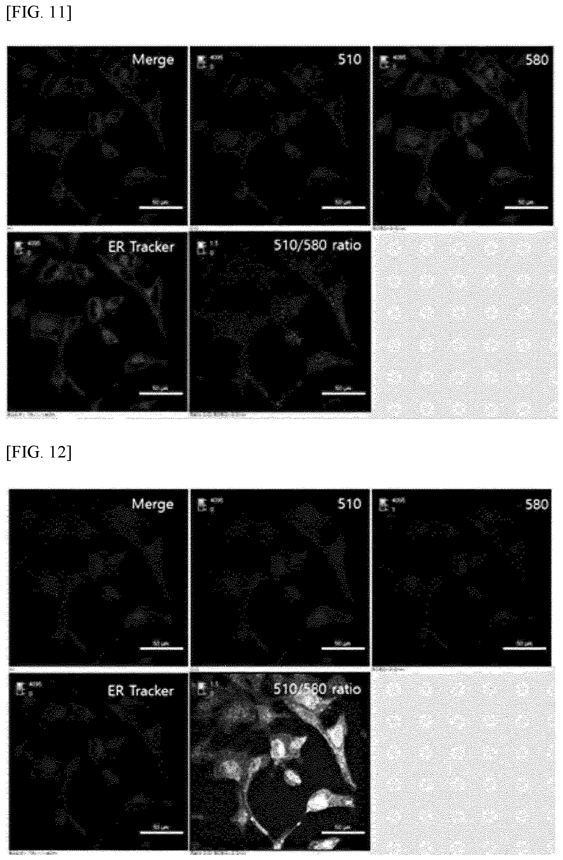

FIG. 11 shows confocal microscope images of the endoplasmic reticulum of UC-MSCs to which diamide was added after treatment with the ER-FT represented by Formula V.

FIG. 12 shows confocal microscope images of the endoplasmic reticulum of UC-MSCs to which diamide and DTT were added after treatment with the ER-FT represented by Formula V.

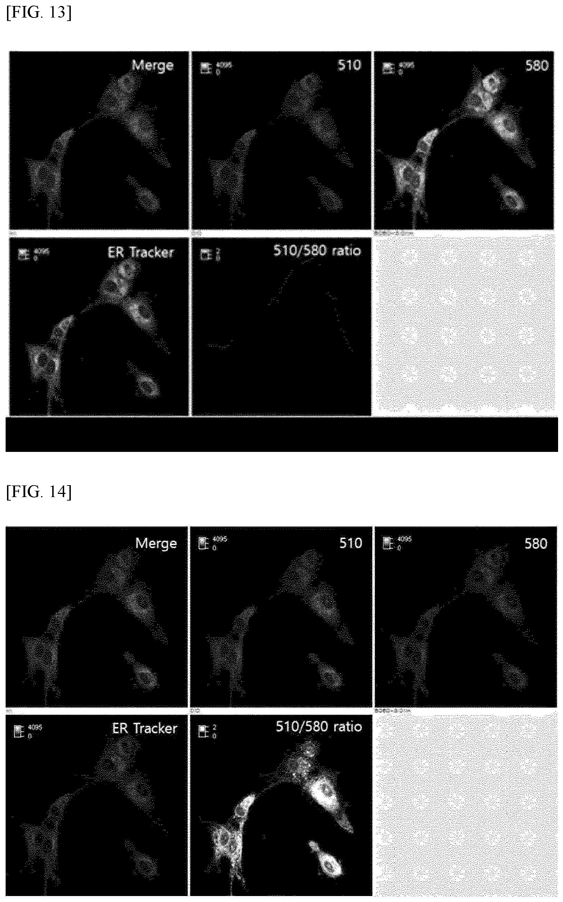

FIG. 13 shows confocal microscope images of the endoplasmic reticulum of UC-MSCs to which diamide was added after treatment with the ER-FT represented by Formula VI.

FIG. 14 shows confocal microscope images of the endoplasmic reticulum of UC-MSCs to which diamide and DTT were added after treatment with the ER-FT represented by Formula VI.

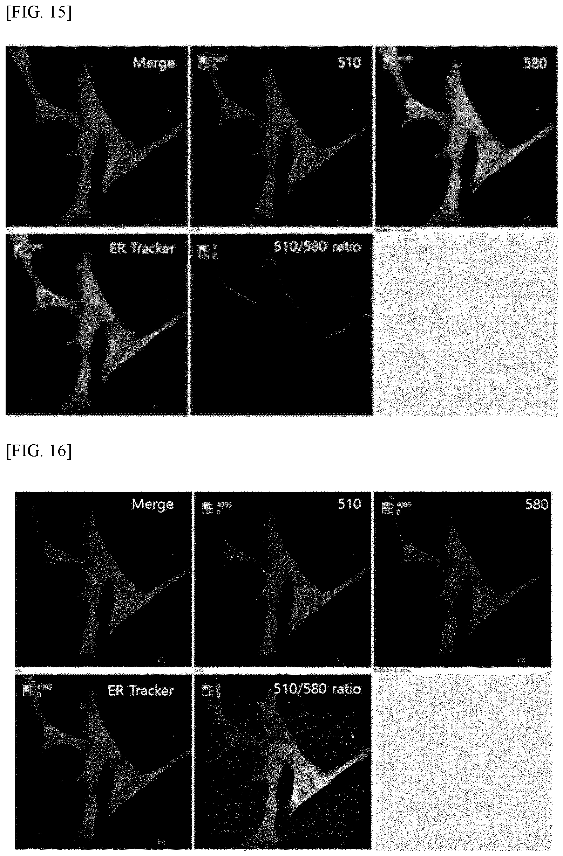

FIG. 15 shows confocal microscope images of the endoplasmic reticulum of UC-MSCs to which diamide was added after treatment with the ER-FT represented by Formula VII.

FIG. 16 shows confocal microscope images of the endoplasmic reticulum of UC-MSCs to which diamide and DTT were added after treatment with the ER-FT represented by Formula VII.

(1) Cells (UC-MSC: 5×10⁴) were seeded into confocal dishes and then cultured for 24 hours. (2) 2 ml of each cell type medium containing each of ER-FTs (Formulas IV to VI) at a concentration of 10 to 20 µM was prepared. (3) After medium suction from the dishes, ER-FT staining was performed, followed by incubation in an incubator at 37°C for 30 minutes. (4) 2 µl of 1 mM ER tracker Red was added to each medium and mixed well, followed by incubation for 30 minutes. (5) After medium suction, 2 ml of HBSS was added. (6) After the ER targeting experiment, 10 µl of 100 mM diamide was pipetted into the cells where the laser did hit. (7) Changes in the cells were observed through confocal imaging. (8) 20 µl of 1M DTT was pipetted in the same way as above. (9) Changes in the cells were observed through confocal imaging.

As shown in FIGS. 9 to 16, the four types of ER-FTs (Formulas IV to VII) all showed a decrease in the fluorescence intensity at 510 nm and an increase in the fluorescence intensity at 580 nm when treated with diamide, and showed an increase in the fluorescence intensity at 510 nm and a decrease in the fluorescence intensity at 580 nm when treated with DTT.

Using the compound or composition according to the present invention, it is possible to measure the antioxidant activities of the endoplasmic reticulum (ER) and Golgi apparatus, which are organelles in living cell. When the compound or composition is applied to stem cells, it is possible to screen highly active stem cells based on the results of measurement of antioxidant activity in stem cells, thereby increasing the efficiency of cell therapy products.

All the references, articles, publications, patents and patent applications cited in this specification are incorporated herein in their entirety. Therefore, the spirit and scope of the appended claims should not be limited to the description of the preferred embodiments disclosed herein.

### Industrial Applicability

The present invention relates to a real-time fluorescence imaging sensor for measuring glutathione in the endoplasmic reticulum (ER) and a method using the same. More specifically, the present invention relates to a novel compound for measuring glutathione in the endoplasmic reticulum (ER) and a method of measuring glutathione in the endoplasmic reticulum (ER) using the novel compound.

## Claims

1. A compound represented by the following Formula I or a pharmaceutically acceptable salt thereof: 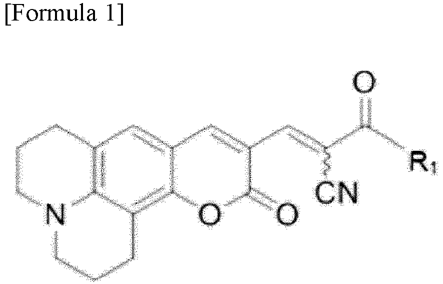 wherein R₁ is a 3- to 7-membered heterocycloalkyl ring containing at least one N atom, an R₂ substituent is bonded to the heterocycloalkyl, and R₂ is an amide group.

2. The compound or pharmaceutically acceptable salt thereof according to claim 1, wherein R₂ is -(C(=O)NH)-R₃, wherein R₃ is -(CH₂)ₘ-R₄, -(CH₂)ₘ-R₅, -(CH₂)ₙ-(CH₂₀CH₂)ₚ-(CH₂)_{q}-R₄, or -(CH₂)ₙ-(CH₂₀CH₂)ₚ-(CH₂)_{q}-R₅, wherein m is an integer ranging from 1 to 6, n, p and q are each independently an integer ranging from 1 to 4, R₄ is a substituent represented by the following Formula II, and R₅ is a substituent represented by the following Formula III:

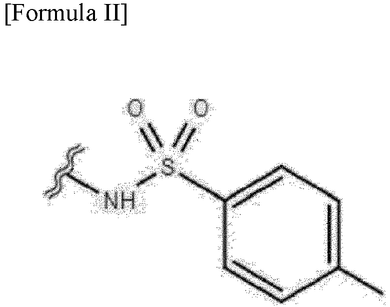

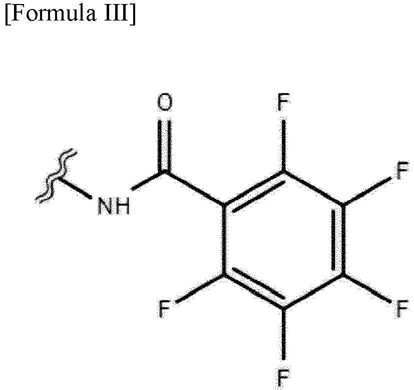

3. The compound or pharmaceutically acceptable salt thereof according to claim 2, wherein the compound represented by Formula I is represented by any one of the following Formulas IV to VII:

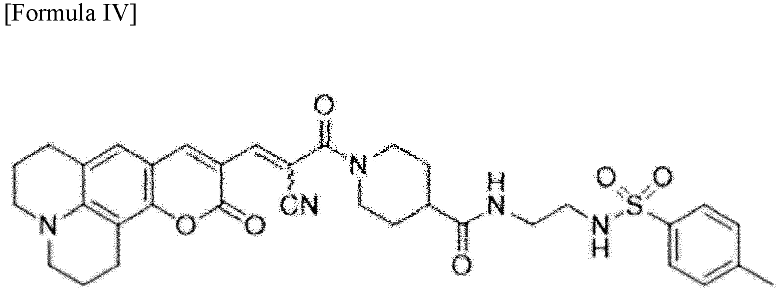

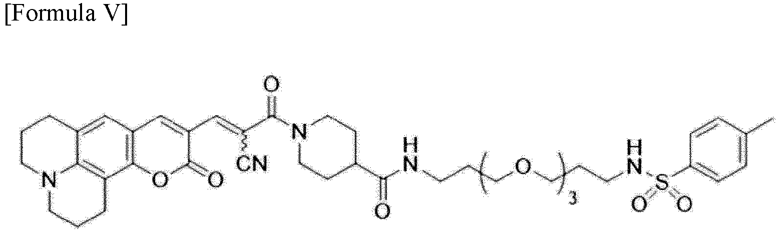

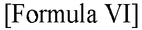

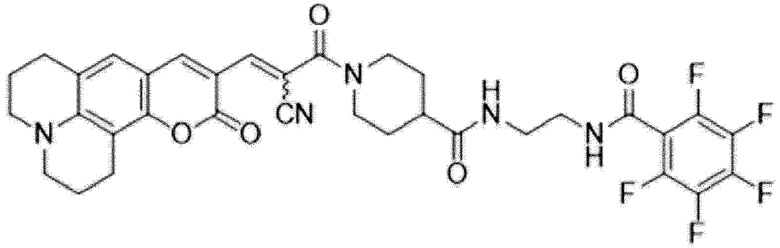

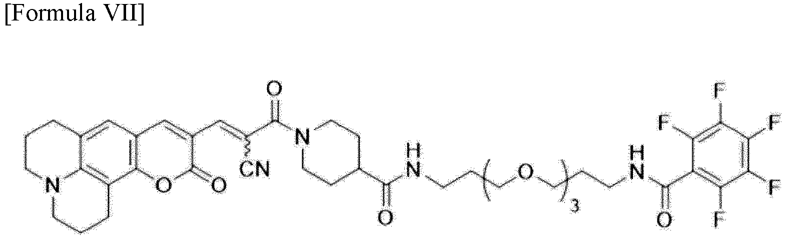

4. The compound or pharmaceutically acceptable salt thereof according to claim 1, wherein the compound represented by Formula I shows a maximum emission wavelength at 550 to 680 nm in a free state, and shows a maximum emission wavelength at 430 to 550 nm in a thiol-bound state.

5. The compound or pharmaceutically acceptable salt thereof according to claim 4, wherein the fluorescence intensity increases or decreases at an emission wavelength ranging from 430 nm to 680 nm.

6. A composition for measurement of antioxidant activity in living cells, the composition containing, as an active ingredient, the compound or pharmaceutically acceptable salt thereof according to any one of claims 1 to 5.

7. The composition according to claim 6, wherein the measurement of the antioxidant activity is measurement of the level of thiols in the living cells.

8. The composition according to claim 7, wherein the measurement of the level of thiols is measurement of the level of thiols in a cell organelle in the living cells.

9. The composition according to claim 8, wherein the cell organelle is endoplasmic reticulum (ER).

10. The composition according to claim 9, wherein the measurement of the antioxidant activity in the endoplasmic reticulum (ER) is performed using the compound of any one of Formulas IV to VII.

11. The composition according to claim 7, wherein, as the level of thiols in the measurement of the level of thiols increases, the fluorescence intensity at 550 to 680 nm decreases and the fluorescence intensity at 430 to 550 nm increases.

12. The composition according to claim 7, wherein the measurement of the level of thiols is performed by obtaining a ratio of the fluorescence intensity at 430 to 550 nm to the fluorescence intensity at 550 to 680 nm.

13. The composition according to claim 12, wherein the ratio is a relationship between the fluorescence intensity at 430 to 550 nm and the fluorescence intensity at 550 to 680 nm.

14. The composition according to claim 13, wherein the relationship is a mathematical ratio between the fluorescence intensity at 430 to 550 nm and the fluorescence intensity at 550 to 680 nm, wherein the mathematical ratio increases or decreases ratiometrically and reversibly depending on the amount of thiols in the endoplasmic reticulum (ER), thereby indicating the amount of thiols in the cell organelle in real time.

15. The composition according to claim 7, wherein the measurement of the level of thiols is quantitative or qualitative detection of the thiols in the endoplasmic reticulum (ER).

16. The composition according to claim 7, wherein the measurement of the level of thiols is real-time quantitative measurement.

17. The composition according to claim 7, wherein the measurement of the level of thiols indicates the oxidative stress or degree of oxidation of the cells.

18. The composition according to claim 7, wherein the measurement of the level of thiols indicates the degree of senescence of the cells.

19. The composition according to claim 7, wherein the thiols are glutathione (GSH), homocysteine (Hcy), cysteine (Cys), or thiols in cysteine residues of proteins.

20. A method for screening a thiol enhancer or inhibitor in living cells, the method comprising steps of:
(a) adding the composition of claim 6 and a candidate substance simultaneously or sequentially in any order to living cells;
(b) obtaining a ratio of the fluorescence intensity of the living cells at 430 to 550 nm to the fluorescence intensity of the living cells at 550 to 680 nm and comparing the obtained ratio with standard data;
(c) determining that the candidate substance is a thiol enhancer or inhibitor; and
(d) determining that when the ratio of the fluorescence intensity at 550 to 680 nm to the fluorescence intensity at 430 to 550 nm decreases, the candidate substance is the thiol enhancer, and determining that when the ratio of the fluorescence intensity at 550 to 680 nm to the fluorescence intensity at 430 to 550 nm increases, the candidate substance is the thiol inhibitor.

21. The method according to claim 20, wherein step (d) comprises determining that when a 510/580 ratio, which is the ratio of the fluorescence intensity at 510 nm to the fluorescence intensity at 580 nm, decreases, the candidate substance is the thiol inhibitor, and when the 510/580 ratio, which is the ratio of the fluorescence intensity at 510 nm to the fluorescence intensity at 580 nm, increases, the candidate substance is the thiol enhancer.

22. The method according to claim 20, wherein the ratio of the fluorescence intensities is obtained for endoplasmic reticulum (ER).

23. A kit for diagnosing oxidative stress-induced disease comprising the compound or salt thereof according to any one of claims 1 to 5.

24. A method for measuring antioxidant activity in living cells, the method comprising steps of:
(a) measuring in real time the ratio of the fluorescence intensity of the living cells at 430 to 550 nm to the fluorescence intensity of the living cells at 550 to 680 nm;
(b) adding the composition of claim 6 to the living cells;
(c) adding an oxidizing agent to the living cells of step (b); and
(d) observing a change in the ratio of the fluorescence intensities.

25. The method according to claim 24, wherein step (a) comprises measuring in real time a 510/580 ratio which is the ratio of the fluorescence intensity at 510 nm to the fluorescence intensity at 580 nm.

26. The method according to claim 24, further comprising, after step (d), a step of measuring the time for the ratio of the fluorescence intensities to return to either the fluorescence intensity ratio of the living cells to which the oxidizing agent was not added or the fluorescence intensity ratio shown before the oxidizing agent is added, wherein it is determined that the shorter the time, the higher the antioxidant activity.

27. The method according to claim 24, further comprising, after step (d), a step of measuring the integrated value of the difference between the fluorescence intensity ratio of the living cells to which the oxidizing agent was not added and the fluorescence intensity ratio of the living cells to which the oxidizing agent was added, from a time point at which the oxidizing agent was added to a time point at which the fluorescence intensity ratio returns to the fluorescence intensity ratio shown before the oxidizing agent is added, wherein it is determined that the smaller the integrated value, the higher the antioxidant activity.

28. The method according to claim 24, further comprising, after step (d), a step of determining the minimum concentration of the oxidizing agent, at which the fluorescence intensity ratio of the living cells to which the oxidizing agent was added starts to decrease, wherein it is determined that the higher the minimum concentration, the higher the antioxidant activity.

29. The method according to claim 24, wherein the measuring is performed for endoplasmic reticulum (ER) which is an organelle in the living cells.
